# FASCICULE DE BREVET EUROPEEN

(11) **EP 2 959 312 B1**
(45) Date de publication et mention de la délivrance du brevet: **13.12.2017**
(21) Numéro de dépôt: 14705516.4
(22) Date de dépôt: 19.02.2014
(51) Int. Cl.: G01S 7/52, A61B 8/08

(54) **PROCEDE D'ELASTOGRAPHIE MULTI-IMPULSIONNELLE**
MULTIPULS-ELASTOGRAFIEVERFAHREN
MULTIPULSE ELASTOGRAPHY METHOD

(30) Priorité: 19.02.2013 FR 1351405
(43) Date de publication de la demande: 30.12.2015
(73) Titulaire: Echosens, 75013 Paris (FR)
(72) Inventeur: SANDRIN, Laurent, F-92240 L'Hay Les Roses (FR); BASTARD, Cécile, F-75012 Paris (FR)
(74) Mandataire: Cabinet Camus Lebkiri
(86) Numéro de dépôt international: PCT/EP2014/053264
(87) Numéro de publication internationale: WO 2014/128182

(56) Documents cités:
- EP-A1- 2 294 983
- WO-A1-2011/064688
- WO-A1-2011/132014
- WO-A2-2011/007278
- US-A1- 2010 069 751
- US-A1- 2010 256 530
- US-A1- 2012 158 323

## Description

### DOMAINE TECHNIQUE DE L'INVENTION

La présente invention concerne un procédé d'élastographie multi-impulsionnelle pour la mesure quantitative d'au moins une propriété mécanique d'un milieu viscoélastique présentant un signal ultrasonore après illumination ultrasonore. Dans une application non limitative, l'invention concerne un procédé d'élastographie multi-impulsionnelle pour la mesure quantitative de l'élasticité et de la viscosité d'un organe humain ou animal, par exemple le tissu hépatique.

### ETAT DE LA TECHNIQUE ANTERIEUR

On connaît un procédé pour observer simultanément la propagation d'une onde impulsionnelle de cisaillement basse fréquence en une multitude de points d'un milieu viscoélastique diffusant. A cet effet, on émet à cadence ultrarapide des ondes ultrasonores de compression qui permettent d'obtenir une succession de mesures du milieu, puis on traite en temps différé voire même réel les mesures ainsi obtenues, pour déterminer les mouvements du milieu lors de la propagation de l'onde de cisaillement.

La demande de brevet FR2843290 décrit un dispositif pour la mesure de l'élasticité d'un organe présentant un signal ultrasonore après illumination ultrasonore, le dispositif comprenant un transducteur ultrasonore, et un actionneur électrodynamique asservi apte à faire vibrer à basse fréquence le transducteur pour émettre une onde de cisaillement dans le tissu. L'onde de cisaillement présente une bande fréquentielle dont la fréquence centrale est déterminée, les fréquences environnantes de la fréquence centrale sont très atténuées de sorte que des données ne peuvent être obtenues que sur des fréquences très proches de la fréquence centrale. Il s'ensuit que les mesures effectuées ne permettent pas de caractériser entièrement le tissu.

Il existe également une technologie dénommée élastographie par force de radiation ultrasonore (également connue sous l'acronyme ARFI) via laquelle les tissus sont mis en mouvement par l'action d'une force produite par la pression de radiation issue d'un faisceau ultrasonore. Ce déplacement correspond à la génération de contraintes de cisaillement dans les tissus et se manifeste par la propagation d'une onde de cisaillement. Dans le cas de l'ARFI, l'onde de cisaillement est très vite atténuée et se propage généralement sur moins d'une longueur d'onde. Dans ces conditions, il est difficile d'étudier les caractéristiques fréquentielles de l'onde de cisaillement. Les scientifiques qui utilisent cette technique s'intéressent donc généralement à l'étude des temps de montée ou de relaxation et à l'amplitude des déplacements (cf. brevet US 2010/069751 et WO 2011/064688). En modulant le faisceau ultrasonore, c'est-à-dire les émissions ultrasonores, par exemple en terme de fréquence, d'amplitude ou de cadence, il est possible de moduler les déplacements induits dans les tissus. Certaines équipes ont donc proposé d'utiliser successivement plusieurs types d'excitations ultrasonores de propriétés différentes afin de moduler la réponse du tissu en termes de déplacement maximal observé, de temps de montée et de temps de relaxation (cf. brevet US 2010/069751). Cependant, la modulation des paramètres des émissions ultrasonores ne permet pas de moduler avec précision les déplacements des tissus, en particulier leur contenu fréquentiel. Les déplacements générés par la force de pression de radiation dépendent en effets du coefficient d'absorption des tissus.

Dans ce contexte, l'invention vise à proposer un procédé d'élastographie permettant de surmonter les inconvénients de l'état de la technique et vise notamment un procédé d'élastographie permettant d'obtenir rapidement des mesures quantitatives précises des propriétés mécaniques d'un organe humain ou animal.

### RESUME DE L'INVENTION

A cette fin, l'invention porte sur un procédé d'élastographie multi-impulsionnelle pour la mesure quantitative d'au moins une propriété mécanique d'un milieu viscoélastique présentant un signal ultrasonore après illumination ultrasonore, ledit procédé comportant les étapes suivantes :
- définition des caractéristiques d'au moins deux impulsions mécaniques basses fréquences, présentant une fréquence centrale comprise entre 10 Hz et 5000 Hz,
- génération, via un actionneur électrodynamique, desdites au moins deux impulsions mécaniques,
- suivi de la propagation dans un milieu viscoélastique d'au moins deux ondes de cisaillement produites par lesdites au moins deux impulsions mécaniques au moyen d'émission et acquisition de signaux ultrasonores,
- calcul d'au moins une propriété mécanique dudit milieu viscoélastique au moyen desdites acquisitions desdits signaux ultrasonores.

Dans une mise en oeuvre non limitative, pour chacune des impulsions mécaniques au moins une des caractéristiques définies est différente. De façon non limitative, on entend par caractéristique de l'impulsion mécanique, une fréquence centrale, une amplitude, un nombre de périodes et/ou un profil temporel déterminé.

L'invention proposée permet de réaliser une acquisition d'élastographie impulsionnelle composée d'une série d'impulsions présentant par exemple des caractéristiques différentes, telles que des fréquences centrales déterminées différentes. Dans cet exemple non limitatif, chaque impulsion permet d'étudier une bande fréquentielle située autour de sa fréquence centrale déterminée. Cette particularité permet de caractériser le milieu sur une gamme fréquentielle importante.

Outre les caractéristiques qui viennent d'être évoquées dans le paragraphe précédent, le procédé selon l'invention peut présenter une ou plusieurs caractéristiques complémentaires parmi les suivantes, considérées individuellement ou selon toutes les combinaisons techniquement réalisables.

Dans une mise en oeuvre non limitative du procédé d'élastographie multi-impulsionnelle, le procédé comporte une étape de réitération de l'étape de génération, de l'étape de suivi et de l'étape de calcul.

Dans une mise en oeuvre non limitative du procédé d'élastographie multi-impulsionnelle, la réitération est réalisée entre 1 et 1000 fois, de préférence entre 1 et 20 fois.

Dans une mise en oeuvre non limitative du procédé d'élastographie multi-impulsionnelle, la au moins une caractéristique différente est l'amplitude.

Dans une mise en oeuvre non limitative du procédé d'élastographie multi-impulsionnelle, chaque impulsion mécanique présente une amplitude comprise entre 10 µm et 10 mm, de préférence entre 100 µm et 5 mm.

Dans une mise en oeuvre non limitative du procédé d'élastographie multi-impulsionnelle, la au moins une caractéristique différente est le profil temporel et/ou le nombre de périodes.

Dans une mise en oeuvre non limitative du procédé d'élastographie multi-impulsionnelle, la au moins une caractéristique différente est la fréquence centrale.

Dans une mise en oeuvre non limitative du procédé d'élastographie multi-impulsionnelle, chaque impulsion mécanique succédant à une impulsion mécanique présente une fréquence centrale inférieure à la fréquence centrale de l'impulsion mécanique qui la précède.

Dans une mise en oeuvre non limitative du procédé d'élastographie multi-impulsionnelle, chaque impulsion mécanique succédant à une impulsion mécanique présente une fréquence centrale supérieure à la fréquence centrale de l'impulsion mécanique qui la précède.

Dans une mise en oeuvre non limitative du procédé d'élastographie multi-impulsionnelle, les bandes fréquentielles d'au moins deux impulsions mécaniques se chevauchent partiellement.

Dans une mise en oeuvre non limitative du procédé d'élastographie multi-impulsionnelle, chaque impulsion mécanique présente une fréquence centrale comprise entre 20 Hz et 1000 Hz.

L'invention porte également sur un dispositif d'élastographie multi-impulsionnelle comportant un générateur de vibration apte à générer une pluralité d'impulsions mécaniques, chaque impulsion mécanique générant une onde de cisaillement dans un milieu viscoélastique, et au moins un transducteur ultrasonore apte à émettre et acquérir des signaux ultrasonores, ledit dispositif étant caractérisé en ce qu'il est apte à mettre en oeuvre les étapes du procédé d'élastographie multi-impulsionnelle selon l'invention.

Dans une mise en oeuvre non limitative, le générateur de vibration est un actionneur électrodynamique asservi et est apte à faire vibrer à basse fréquence le transducteur (cette vibration est une impulsion mécanique) pour émettre une onde de cisaillement dans le tissu. La présente invention propose donc de modifier les caractéristiques de la commande de cet actionneur électrodynamique (donc de l'impulsion mécanique) afin de moduler précisément les caractéristiques de l'onde de cisaillement générée et en particulier son contenu fréquentiel. Les caractéristiques d'une onde de cisaillement générées par une impulsion mécanique dont les propriétés sont connues peuvent être calculées par exemple au moyen des fonctions de Green élastodynamiques.

### BREVE DESCRIPTION DES FIGURES

D'autres caractéristiques et avantages de l'invention ressortiront clairement de la description qui en est donnée ci-dessous, à titre indicatif et nullement limitatif, en référence aux figures annexées, parmi lesquelles :
- la figure 1 illustre un synoptique des étapes d'un procédé d'élastographie multi-impulsionnelle selon l'invention ;
- la figure 2 illustre, de façon schématique, un exemple de réalisation d'un dispositif d'élastographie multi-impulsionnelle selon l'invention ;
- La figure 3 illustre trois impulsions mécaniques présentant chacune une fréquence centrale différente, les trois impulsions mécaniques ayant été générées via le procédé selon l'invention ;
- La figure 4 illustre des impulsions mécaniques présentant un nombre de périodes différent, les impulsions mécaniques ayant été générées par le procédé selon l'invention ;
- La figure 5 illustre une réitération des trois impulsions mécaniques qui sont illustrées sur la figure 3.

Dans toutes les figures, les éléments communs portent les mêmes numéros de référence.

### DESCRIPTION DETAILLEE D'AU MOINS UN MODE DE REALISATION NON LIMITATIF DE L'INVENTION

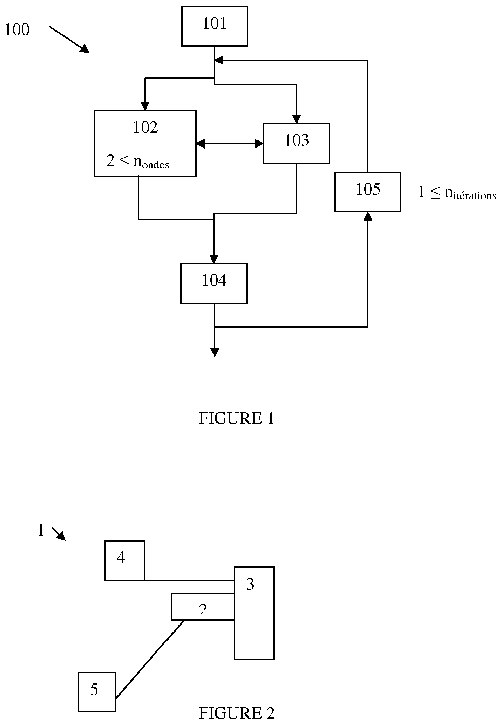

La figure 1 illustre un synoptique des étapes du procédé d'élastographie multi-impulsionnelle 100 selon l'invention.

Le procédé d'élastographie multi-impulsionnelle 100 comporte notamment une étape de définition 101 des caractéristiques d'au moins deux impulsions mécaniques basse fréquence. Dans cet exemple, pour chacune des impulsions mécaniques au moins une des caractéristiques définies est différente.

De façon non limitative, une impulsion mécanique peut être caractérisée par une fréquence centrale, une amplitude, un nombre de périodes et/ou un profil temporel déterminé.

Ainsi, lors de cette étape de définition 101 un opérateur peut par exemple définir un profil temporel de type rampe pour une première impulsion, un profil temporel de type sinus apodisé pour une deuxième impulsion, un profil temporel de type créneau pour une troisième impulsion, un profil temporel de type gaussienne pour une quatrième impulsion et un profil temporel de type sinusoïdal pour une cinquième impulsion.

A titre illustratif, un profil temporel de type sinusoïdal peut être défini par la formule suivante : S(t) = A sin(2π*f*t)
Où :
- t∈ [0 n T]
- T est la période du signal, T= 1/*f*
- n est le nombre de périodes, et
- A est l'amplitude.

Dans le cas d'impulsions mécaniques présentant un profil temporel de type sinusoïdal, il est possible de faire varier la fréquence, le nombre de périodes et/ou l'amplitude. A titre d'exemple, chaque impulsion mécanique peut présenter une amplitude comprise entre 10 µm et 10 mm, de préférence entre 100 µm et 5 mm.

En résumé, lors de cette étape 101 de définition, chaque impulsion mécanique présente une caractéristique différente par rapport aux autres impulsions mécaniques définies. En d'autres termes, chacune des impulsions mécaniques définies est différente des autres impulsions mécaniques définies.

Le procédé d'élastographie multi-impulsionnelle 100 comporte en outre une étape de génération 102 des au moins deux impulsions mécaniques définies lors de l'étape 101 de définition précédente, les au moins deux impulsions mécaniques générant chacune une onde de cisaillement dans un milieu viscoélastique de façon à ce que cette onde se propage au travers de ce milieu viscoélastique.

Ces impulsions mécaniques (indifféremment dénommées impulsions basses fréquences) peuvent être générées au moyen d'un vibreur basse fréquence, d'un haut-parleur ou tout autre type de générateur de vibration 2 (Cf. figure 2) apte à générer une pluralité d'impulsions mécaniques, chaque impulsion mécanique générant une onde de cisaillement basse fréquence dans un milieu viscoélastique, tel qu'un tissu biologique humain ou animal. Le déclenchement de l'étape de génération 102 peut être effectué automatiquement ou manuellement. Un déclenchement manuel est effectué par une pression exercée par un opérateur sur un bouton de déclenchement tandis qu'un déclenchement automatique peut simplement être réalisé dès lors qu'une pression est exercée du milieu viscoélastique vers le générateur de vibration.

Ces impulsions mécaniques présentent chacune une fréquence centrale déterminée. La fréquence centrale déterminée de ces impulsions mécaniques est choisie entre une fréquence minimale qui est par exemple de 10 Hz et une fréquence maximale qui est par exemple de 5000Hz.

Le procédé d'élastographie multi-impulsionnelle 100 comporte également une étape de suivi 103 de la propagation dans le milieu viscoélastique des au moins deux ondes de cisaillement. Ce suivi 103 étant réalisé au moyen d'émission de signaux ultrasonores dans le milieu viscoélastique et d'acquisition de signaux ultrasonores réfléchis par le milieu viscoélastiques.

Cette étape de suivi 103 est réalisée au moyen d'un transducteur ultrasonore 3 mono-élément ou multiéléments.

Dans un exemple non limitatif, chaque impulsion mécanique présente une fréquence centrale déterminée différente, autrement dit une période différente.De ce fait, chaque impulsion mécanique présente une bande fréquentielle différente, cette bande fréquentielle étant formée par la fréquence centrale de l'impulsion mécanique et les fréquences environnantes de la fréquence centrale.

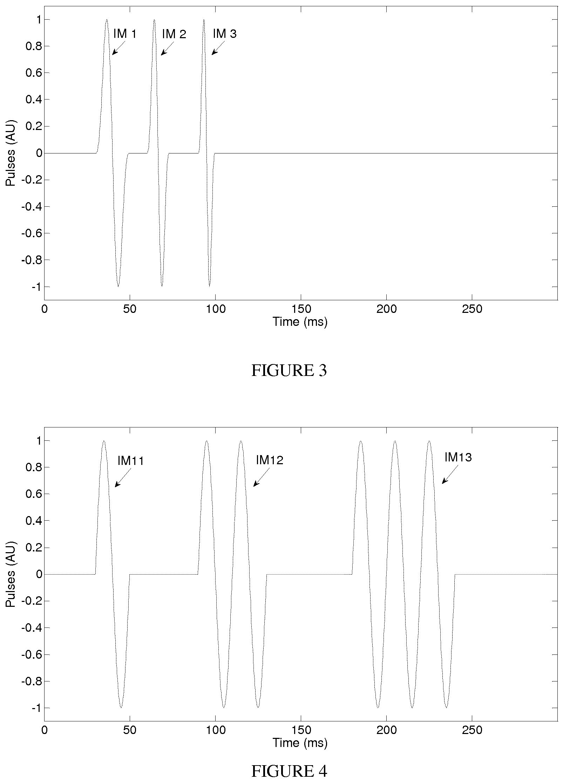

A titre d'exemple non limitatif illustré sur la figure 3, lors de la mise en oeuvre du procédé selon l'invention sur le tissu hépatique :
- une première onde de cisaillement est générée via unepremière impulsion mécanique IM1 produite par le vibreur basse fréquence 2, cette première impulsion mécanique IM1 présentant une fréquence centrale déterminée de 50Hz ;
- une deuxième onde de cisaillement est générée via le vibreur basse fréquence 2, cette deuxième onde de cisaillement est issue d'une deuxième impulsion mécanique IM2 présentant une fréquence centrale déterminée de 75Hz;
- une troisième onde de cisaillement est générée via le vibreur basse fréquence 2, cette troisième onde de cisaillement est issue d'une troisième impulsion mécanique IM3 présentant une fréquence centrale déterminée de 100Hz.

Dans cet exemple, les bandes fréquentielles des impulsions mécaniques IM1, IM2 et IM3 à l'origine des ondes de cisaillement se chevauchent partiellement. Plus précisément, la bande fréquentielle de la première impulsion mécanique IM1 générant la première onde de cisaillement chevauche partiellement la bande fréquentielle de la deuxième impulsion mécanique IM2 générant la deuxième onde de cisaillement, et la bande fréquentielle de la deuxième impulsion mécanique IM2 générant la deuxième onde de cisaillement chevauche partiellement la bande fréquentielle de la troisième impulsion mécanique IM3 générant la troisième onde de cisaillement. De ce fait, la bande fréquentielle totale est formée par la somme des trois bandes fréquentielles. Cette bande fréquentielle totale permet de caractériser le tissu hépatique plus précisément qu'une bande fréquentielle plus réduite.

Dans cet exemple, chaque impulsion mécanique (impulsion mécanique IM2 générant la deuxième onde de cisaillement) succédant à une impulsion mécanique (impulsion mécanique IM1 générant la première onde de cisaillement) présente une fréquence centrale supérieure à la fréquence centrale de 'impulsion mécanique qui la précède (impulsion mécanique IM1 générant la première onde de cisaillement).

Il convient de noter que cette mise en oeuvre n'est pas limitative, ainsi dans une mise en oeuvre différente non illustrée du procédé d'élastographie multi-impulsionnelle 100 selon l'invention, chaque impulsion mécanique (impulsion mécanique IM2 générant la deuxième onde de cisaillement) succédant à une impulsion mécanique (impulsion mécanique IM1 générant la première onde de cisaillement) présente une fréquence centrale inférieure à la fréquence centrale de l'impulsion mécanique qui la précède (impulsion mécanique IM1 générant la première onde de cisaillement). Cette mise en oeuvre permet avantageusement que deux ondes de cisaillement qui se succèdent ne se perturbent pas mutuellement. En effet, plus la fréquence est élevée et plus l'onde de cisaillement s'atténue rapidement. Ainsi, si la première impulsion IM1 générant la première onde de cisaillement présente une fréquence centrale de l'ordre de 100Hz, c'est-à-dire supérieure à la fréquence centrale (75Hz) de la deuxième impulsion IM2 générant la deuxième onde de cisaillement, alors la première onde de cisaillement sera atténuée plus rapidement que la deuxième onde de cisaillement réduisant ainsi le risque de perturbation entre les deux ondes de cisaillement.

Dans une mise en oeuvre différente illustrée sur la figure 4, chaque impulsion mécanique définie peut présenter un nombre de périodes différent. Par exemple, la première impulsion IM11 présente une période, la deuxième impulsion IM12 présente deux périodes, et la troisième impulsion IM13 présente trois périodes.

Le procédé d'élastographie multi-impulsionnelle 100 comporte en outre une étape de calcul 104 d'au moins une propriété mécanique du milieu viscoélastique au moyen des acquisitions des signaux ultrasonores. Cette étape de calcul peut être réalisée dès lors que l'étape 103 est terminée.

Dans une mise en oeuvre non limitative, le procédé d'élastographie multi-impulsionnelle 100 comporte en outre une étape de réitération 105 consistant à réitérer au moins une fois les étapes de génération 102, de suivi 103 et de calcul 104.

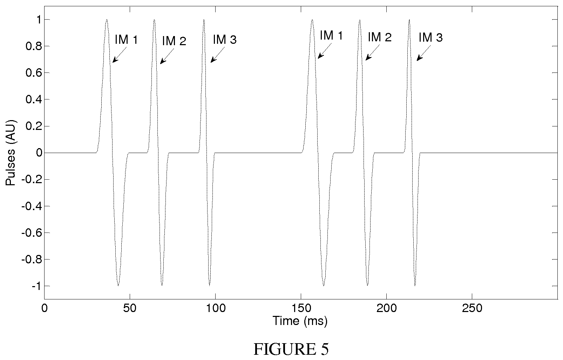

A titre d'exemple illustré sur la figure 5, la réitération 105 de l'étape de génération 102 est effectuée 1 fois. Dans ce cas, les trois impulsions et donc les trois ondes de cisaillement sont générées deux fois dans le milieu viscoélastique.

Dans une mise en oeuvre différente, la réitération 105 est effectuée 20 fois. Lorsque la caractéristique qui varie pour chaque impulsion générant l'onde de cisaillement est la fréquence, un nombre de fois limité permet de couvrir une bande fréquentielle suffisante pour caractériser un tissu biologique tout en étant limité dans le temps de façon à éviter que le tissu biologique ne bouge, par exemple du fait des mouvements biologiques internes tels que la respiration.

Dans une mise en oeuvre non limitative, la réitération 105 des étapes 102, 103 et 104 de génération, de suivi et de calcul est déclenchée de façon automatique. En d'autres termes, l'opérateur qui utilise le dispositif d'élastographie multi-impulsionnelle 1 mettant en oeuvre le procédé 100 selon l'invention n'a pas besoin de déclencher l'étape de réitération 105, cette étape 105 étant déclenchée de façon automatique. En d'autres termes, le nombre de réitérations 105 peut être prédéfini par l'opérateur préalablement au déclenchement du procédé d'élastographie multi-impulsionnelle 100.

Dans une mise en oeuvre différente, la réitération 105 des étapes de génération, de suivi et de calcul est déclenchée de façon manuelle, autrement dit par l'opérateur.

L'invention proposée permet donc de réaliser une acquisition d'élastographie impulsionnelle de type élastographie impulsionnelle à vibration contrôlée (également connue sous l'acronyme VCTE) composée d'une série d'impulsions réalisées, par exemple, à des fréquences centrales différentes comprises entre une fréquence minimale et une fréquence maximale. Chaque impulsion permet par exemple d'étudier une bande fréquentielle située autour de sa fréquence centrale. En juxtaposant les résultats obtenus sur chaque bande fréquentielle, une caractérisation complète du milieu sur la gamme formée de la fréquence minimale à la fréquence maximale est obtenue.

Le procédé 100 selon l'invention permet notamment de :
- Explorer une large gamme fréquentielle (caractérisation complète du milieu),
- contrôler les fréquences utilisées,
- combiner les informations reçues à plusieurs fréquences distinctes,
- réaliser un examen rapide et peu coûteux (versus IRM),
- ne pas déplacer la sonde munie du transducteur entre les différentes acquisitions (réduction de la variabilité du point de mesure),
- faire varier les fréquences limites minimale et maximale selon le milieu étudié,
- faire varier le nombre de périodes,
- faire varier l'amplitude des ondes de cisaillement,
- faire varier la forme de l'impulsion.

L'invention porte également sur un dispositif d'élastographie multi-impulsionnelle 1 comportant un générateur de vibration 2 apte à générer une pluralité d'impulsions mécaniques engendrant une pluralité d'ondes de cisaillement et au moins un transducteur ultrasonore 3 apte à émettre et acquérir des signaux ultrasonores. Le dispositif 1 est apte à mettre en oeuvre les étapes du procédé d'élastographie multi-impulsionnelle 100 selon l'invention, autrement dit le dispositif 1 notamment est apte à :
- définir 101, via une interface homme-machine IHM 5, des caractéristiques d'au moins deux impulsions mécaniques, chaque impulsion mécanique produisant une onde de cisaillement, pour chacune des impulsions mécaniques au moins une des caractéristiques définies pouvant être différente ; ces caractéristiques pouvant être rentrées au moyen d'un clavier par un opérateur,
- générer 102, via le générateur de vibration 2, les au moins deux impulsions mécaniques définies engendrant au moins deux ondes de cisaillement dans un milieu viscoélastique,
- suivre 103, via le transducteur ultrasonore 3, la propagation dans le milieu viscoélastique des au moins deux ondes de cisaillement au moyen d'émission et d'acquisition de signaux ultrasonores,
- calculer 104, via un calculateur 4, au moins une propriété mécanique du milieu viscoélastique au moyen des acquisitions des signaux ultrasonores.

Il convient de noter que pour l'ensemble de la description, lors de l'étape de définition 101 des caractéristiques d'au moins deux impulsions mécaniques basse fréquence, chacune des impulsions mécaniques présente au moins une caractéristique différente. Il est entendu que l'invention ne se limite pas à ce mode de mise en oeuvre et peut présenter une étape de définition 101 des caractéristiques d'au moins deux impulsions mécaniques basse fréquence au cours de laquelle les caractéristiques des au moins deux impulsions mécaniques basse fréquence sont identiques. En effet, certains milieux viscoélastiques ont un temps de relaxation (c'est-à-dire un temps de retour à l'équilibre) très long. Dans ce cas, le milieu n'a donc pas le temps de retrouver son équilibre entre les différentes impulsions. Ainsi en étudiant la propagation des ondes de cisaillement générées de manière consécutive par plusieurs impulsions similaires, on peut obtenir des informations sur les propriétés viscoélastiques du milieu.

## Revendications

1. Procédé (100) d'élastographie multi-impulsionnelle pour la mesure quantitative d'au moins une propriété mécanique d'un milieu viscoélastique présentant un signal ultrasonore après illumination ultrasonore, ledit procédé (100) comportant les étapes suivantes :
- définition (101) des caractéristiques d'au moins deux impulsions mécaniques basses fréquences, présentant une fréquence centrale comprise entre 10 Hz et 5000 Hz,
- génération (102) desdites au moins deux impulsions mécaniques basses fréquences dont les caractéristiques sont définies dans un milieu viscoélastique,
- suivi (103) de la propagation dans ledit milieu viscoélastique des au moins deux ondes de cisaillement issues desdites au moins deux impulsions mécaniques basses fréquences au moyen d'émission et acquisition de signaux ultrasonores,
- calcul (104) d'au moins une propriété mécanique dudit milieu viscoélastique au moyen desdites acquisitions desdits signaux ultrasonores.

2. Procédé selon la revendication 1 selon lequel pour chacune des impulsions mécaniques basses fréquences au moins une des caractéristiques définies est différente.

3. Procédé selon l'une quelconque des revendications précédentes comportant une étape de réitération (105) de l'étape de génération (102), de l'étape de suivi (103) et de l'étape de calcul (104).

4. Procédé selon la revendication 3 précédente, selon lequel la réitération (105) est réalisée entre 1 et 1000 fois, de préférence entre 1 et 20 fois.

5. Procédé selon l'une quelconque des revendications précédentes selon lequel au moins une caractéristique différente est l'amplitude.

6. Procédé selon la revendication 5 précédente, selon lequel chaque impulsion mécanique basses fréquences présente une amplitude comprise entre 10 µm et 10 mm, de préférence entre 100 µm et 5 mm.

7. Procédé selon l'une quelconque des revendications précédentes, selon lequel au moins une caractéristique différente est le profil temporel.

8. Procédé selon l'une quelconque des revendications précédentes, selon lequel au moins une caractéristique différente est le nombre de périodes.

9. Procédé selon l'une quelconque des revendications précédentes, selon lequel au moins une caractéristique différente est la fréquence centrale.

10. Procédé selon la revendication 9 précédente, selon lequel chaque impulsion mécanique basses_fréquences succédant à une impulsion mécanique basses fréquences présente une fréquence centrale inférieure à la fréquence centrale de l'impulsion mécanique qui la précède.

11. Procédé selon la revendication 9 selon lequel chaque impulsion mécanique basses fréquences succédant à une impulsion mécanique basses fréquences présente une fréquence centrale supérieure à la fréquence centrale de l'impulsion mécanique qui la précède.

12. Procédé selon l'une quelconque des revendications précédentes, selon lequel les bandes fréquentielles d'au moins deux impulsions mécaniques basses fréquences se chevauchent partiellement.

13. Procédé selon l'une quelconque des revendications précédentes, selon lequel chaque impulsion mécanique basses fréquences présente une fréquence centrale comprise entre 20 Hz et 1000 Hz.

14. Dispositif d'élastographie multi-impulsionnelle (1) comportant un générateur de vibration (2) apte à générer, dans un milieu viscoélastique, une pluralité d'ondes de cisaillement et au moins un transducteur ultrasonore (3) configuré à émettre et acquérir des signaux ultrasonores, ledit dispositif (1) étant **caractérisé en ce qu'**il est apte à mettre en oeuvre les étapes du procédé d'élastographie multi-impulsionnelle (100) selon l'une quelconque des revendications 1 à 13 précédentes.

## Patentansprüche

1. Multi-Impulselastographieverfahren (100) für die quantitative Messung von wenigstens einer mechanischen Eigenschaft eines viskoelastischen Milieus, das ein Ultraschallsignal nach einer Ultraschallanstrahlung aufweist, wobei das genannte Verfahren (100) die folgenden Schritte umfasst:
- Definition (101) der Merkmale von wenigstens zwei mechanischen Niedrigfrequenzimpulsen, die eine zwischen 10 Hz und 5000 Hz inbegriffene zentrale Frequenz aufweisen;
- Generieren (102) der genannten wenigstens zwei mechanischen Niedrigfrequenzimpulse, deren Merkmale in einem viskoelastischen Milieu definiert sind;
- Verfolgung (103) der Verbreitung in dem genannten viskoelastischen Milieu der wenigstens zwei Abscherstrahlen, die aus den genannten wenigstens zwei mechanischen Niedrigfrequenzimpulsen stammen, mittels Ausgabe und Erfassung von Ultraschallsignalen;
- Berechnung (104) wenigstens einer mechanischen Eigenschaft des genannten viskoelastischen Mittels mittels der genannten Erfassungen der genannten Ultraschallsignale.

2. Verfahren gemäß Anspruch 1, gemäß dem für jeden mechanischen Niedrigfrequenzimpuls wenigsten eines der definierten Merkmale unterschiedlich ist.

3. Verfahren gemäß irgendeinem der voranstehenden Ansprüche, umfassend einen Reiterationsschritt (105) des Generierungsschritts (102), des Verfolgungsschritts (103) und des Berechnungsschritts (104).

4. Verfahren gemäß dem voranstehenden Anspruch 3, gemäß dem die Reiteration (105) zwischen 1 und 1000 Mal, bevorzugt zwischen 1 und 20 Mal realisiert ist.

5. Verfahren gemäß irgendeinem der voranstehenden Ansprüche, gemäß dem wenigstens ein unterschiedliches Merkmal die Amplitude ist.

6. Verfahren gemäß dem voranstehenden Anspruch 5, gemäß dem jeder mechanische Niedrigfrequenzimpuls eine zwischen 10 µm und 10 mm, bevorzugt zwischen 100 µm und 5 mm inbegriffene Amplitude aufweist.

7. Verfahren gemäß irgendeinem der voranstehenden Ansprüche, gemäß dem wenigstens ein unterschiedliches Merkmal das zeitliche Profil ist.

8. Verfahren gemäß irgendeinem der voranstehenden Ansprüche, gemäß dem wenigstens ein unterschiedliches Merkmal die Periodenanzahl ist.

9. Verfahren gemäß irgendeinem der voranstehenden Ansprüche, gemäß dem wenigstens ein unterschiedliches Merkmal die zentrale Frequenz ist.

10. Verfahren gemäß dem voranstehenden Anspruch 9, gemäß dem jeder mechanische Niedrigfrequenzimpuls, der auf einen mechanischen Niedrigfrequenzimpuls folgt, eine zentrale Frequenz aufweist, die niedriger ist als die zentrale mechanische Impulsfrequenz, die ihr voransteht.

11. Verfahren gemäß Anspruch 9, gemäß dem jeder mechanische Niedrigfrequenzimpuls, der auf einen mechanischen Niedrigfrequenzimpuls folgt, eine zentrale Frequenz aufweist, die höher ist als die zentrale Frequenz des mechanischen Impulses, der ihr voransteht.

12. Verfahren gemäß irgendeinem der voranstehenden Ansprüche, gemäß dem die Frequenzbänder von wenigstens zwei mechanischen Niedrigfrequenzbändern sich teilweise überlappen.

13. Verfahren gemäß irgendeinem der voranstehenden Ansprüche, gemäß dem jeder mechanische Niedrigfrequenzimpuls eine zwischen 20 Hz und 1000 Hz inbegriffene zentrale Frequenz aufweist.

14. Multi-Impuluselastographievorrichtung (1), umfassend einen Vibrationsgenerator (2), der geeignet ist, in einem viskoelastischen Milieu eine Vielzahl von Abscherwellen und wenigstens einen Ultraschall-Messwandler (3) zu generieren, der konfiguriert ist, um Ultraschallsignale auszugeben und zu erfassen, wobei die genannte Vorrichtung (1) **dadurch gekennzeichnet ist, dass** sie geeignet ist, die Schritte des Multi-Impulselastograhpieverfahrens (100) gemäß irgendeinem der voranstehenden Ansprüche 1 bis 13 umzusetzen.

## Claims

1. Multipulse elastography method (100) for the quantitative measurement of at least one mechanical property of a viscoelastic medium having an ultrasonic signal after ultrasonic illumination, said method (100) comprising the following steps:
- define (101) the characteristics of at least two low frequency mechanical pulses having a central frequency comprised between 10 Hz and 5000 Hz,
- generate (102) said at least two low frequency mechanical pulses for which characteristics are defined in a viscoelastic medium,
- monitor (103) the propagation of the at least two shear waves generated by said at least two low frequency mechanical pulses using ultrasonic signal emission and acquisition means in said viscoelastic medium,
- calculate (104) at least one mechanical property of said viscoelastic medium using said acquisitions of said ultrasonic signals.

2. Method according to claim 1 in which at least one of the defined characteristics is different for each low frequency mechanical pulse.

3. Method according to any one of the previous claims, comprising a step (105) in which the generation step (102), the monitoring step (103) and the calculation step (104) are reiterated.

4. Method according to the previous claim 3, in which the reiteration (105) is made between 1 and 1000 times, and preferably between 1 and 20 times.

5. Method according to any one of the previous claims, in which at least one different characteristic is the amplitude.

6. Method according to the previous claim 5, in which the amplitude of each low frequency mechanical pulse is between 10 µm and 10 mm, and is preferably between 100 µm and 5 mm.

7. Method according to any one of the previous claims, in which at least one different characteristic is the time profile.

8. Method according to any one of the previous claims, in which at least one different characteristic is the number of periods.

9. Method according to any one of the previous claims, in which at least one different characteristic is the central frequency.

10. Method according to the previous claim 9, in which the central frequency of each low frequency mechanical pulse succeeding a low frequency mechanical pulse is less than the central frequency of the preceding mechanical pulse.

11. Method according to claim 9, in which the central frequency of each low frequency mechanical pulse succeeding a low frequency mechanical pulse is more than the central frequency of the preceding mechanical pulse.

12. Method according to any one of the previous claims, in which the frequency bands of at least two low frequency mechanical pulses partially overlap.

13. Method according to any one of the previous claims, in which the central frequency of each low frequency mechanical pulse is between 20 Hz and 1000 Hz.

14. Multipulse elastography device (1) comprising a vibration generator (2) capable of generating a plurality of shear waves in a viscoelastic medium, and at least one ultrasonic transducer (3) capable of emitting and acquiring ultrasonic signals, said device (1) being **characterised in that** it is capable of implementing the steps in the multipulse elastography method (100) according to any one of the previous claims 1 to 13.
